# EUROPEAN PATENT SPECIFICATION

(11) **EP 1 193 561 B1**
(45) Date of publication and mention of the grant of the patent: **02.01.2008**
(21) Application number: 01306952.1
(22) Date of filing: 16.08.2001
(51) Int. Cl.: G03G 7/00, G03G 13/16, A61F 13/15, D06P 5/00

(54) **Fibrous nonwoven sheet printed with given pattern using electrophotographic process**
Faservliesstoff bedruckt mit einem vorgegebenen Muster unter Verwendung eines elektrophotographischen Verfahrens
Structure fibreuse non tissée imprimée avec un dessin donné et fabriquée d'après le procédé élecrophotographique

(30) Priority: 25.08.2000 JP 2000301207
(43) Date of publication of application: 03.04.2002
(73) Proprietor: UNI-CHARM CORPORATION, Shikokuchuo-shi, Ehime-ken (JP)
(72) Inventor: Taniguchi, Hiroaki, c/o Technical Center, Mitoyo-gun, Kagawa-ken 769-1602 (JP)
(74) Representative: Eke, Philippa Dianne

(56) References cited:
- EP-A- 0 962 831
- EP-A- 1 078 620
- WO-A-96/22565
- US-A- 6 007 955

## Description

This invention relates to a fibrous nonwoven sheet printed with a given pattern using an electrophotographic process.

Japanese Patent Application Publication No. 2000-266A describes a body fluid absorbent wearing article including a sheet member printed with a pattern being visible from the outside using an ink jet process. The article disclosed in this Publication requires no stereotyped roll as gravure or flexography requires and allows the sheet member to be quickly printed with various patterns by a printer or a plotter using a so-called ink jet process.

The ink jet process has conventionally used a super-penetrant liquid ink in order to avoid undesirable color bleeding with respect to the sheet member. The Publication describes an example of the sheet member used as the component of the wearing article, which is formed with a plurality of thermoplastic synthetic resin fibers and printed with a pattern using the ink jet process. However, if the super-penetrant ink is used, the ink may permeate the nonwoven fabric through its surface and spread therein until the pattern is made indistinct due to bleeding of the ink and the color tone of the pattern is varied due to mingling of the ink.

It is an object of this invention to provide a fibrous nonwoven sheet printed with a pattern adapted to maintain its distinctness substantially without variation in its color tone.

According to this invention, there is provided a fibrous nonwoven sheet printed with a given pattern using an electrophotographic process comprising the steps of photoconductively forming an electrostatic latent image on a photosensitive body, electrostatically depositing colored toner (charged fine grains) on the electrostatic latent image to convert the electrostatic latent image to the corresponding visible image and transferring the visible image onto a surface of the fibrous nonwoven sheet using an electrostatic transfer process.

The improvement according to this invention is in that the fibrous nonwoven sheet is a plurality of thermoplastic synthetic resin fibers and has a given thickness, the toner has an outer layer slightly permeating the fibrous nonwoven sheet through its surface and deposited around fibers lying in a vicinity of the surface of the fibrous nonwoven sheet and an inner layer deposited around fibers immediately underlying the surface; the toner is in the form of a plurality of independent dots and respective pairs of adjacent dots of the toner are not mingled together; the outer layer of each toner dot has a thickness dimension in a range of 1 to 100 µm; the outer layer of each toner dot exposed on said surface of said fibrous nonwoven sheet has a surface area in a range of 10 to 100 µm² ; and a thickness dimension of each toner dot inclusive of said outer layer and said inner layer is 10 µm or larger and less than a thickness of said nonwoven sheet itself.

According to a preferred embodiment of this invention, the sheet member is used as at least a liquid-impervious backsheet in a disposable body fluid absorbent wearing article comprising a liquid-pervious topsheet, said liquid-impervious backsheet and a liquid-absorbent core disposed between the top- and backsheets.
Fig. 1 is a perspective view of a fibrous nonwoven sheet according to this invention;
Fig. 2 is a fragmentary perspective view showing a part of the fibrous nonwoven sheet of Fig. 1 in an enlarged scale;
Fig. 3 is a sectional view taken along a line A - A in Fig. 2;
Fig. 4 is a diagram illustrating an example of electrophotographic printing process;
Fig. 5 is a perspective view of a disposable diaper using a fibrous nonwoven sheet printed with a pattern; and
Fig. 6 is a perspective view of a package using a fibrous nonwoven sheet printed with the pattern.

Details of a fibrous nonwoven sheet according to this invention will be more fully understood from the description given hereunder with reference to the accompanying drawings.

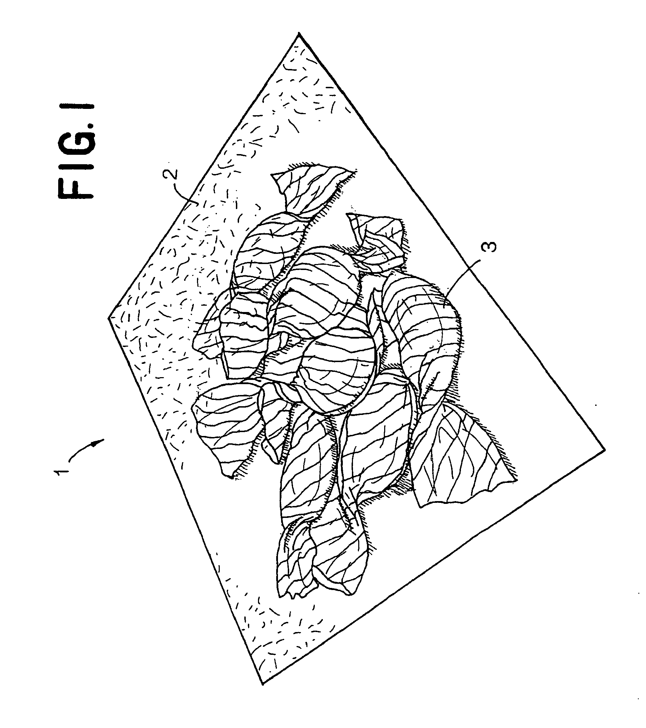

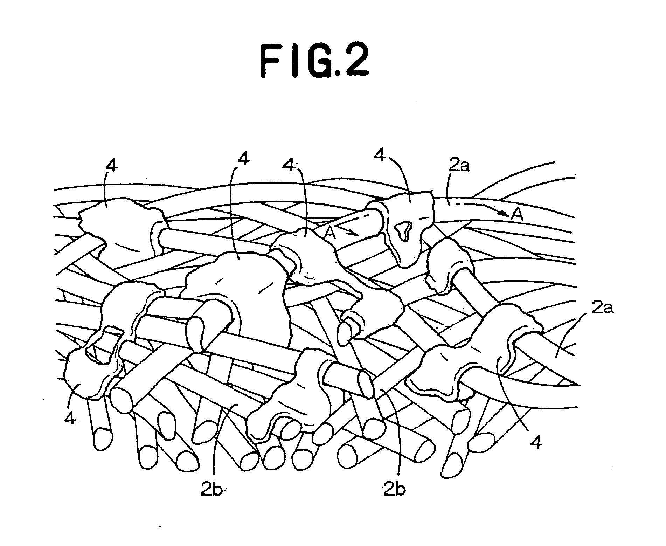

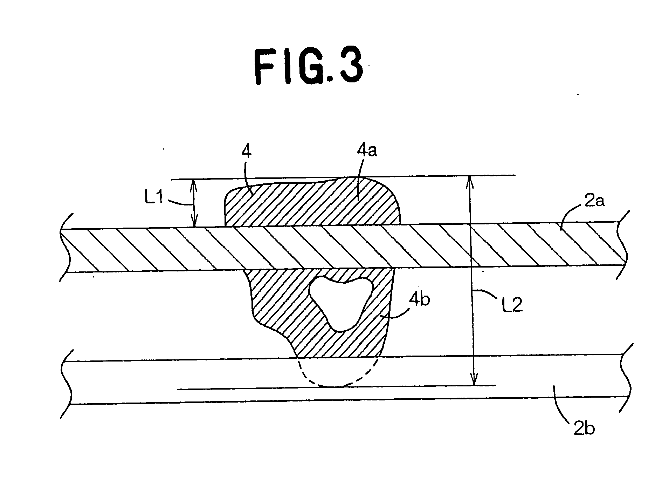

Fig. 1 is a perspective view of a sheet member 1, Fig. 2 is a fragmentary perspective view showing a part of a fibrous nonwoven sheet (fabric) 1 of Fig. 1 in an enlarged scale and Fig. 3 is a sectional view taken along a line A - A in Fig. 2. The fibrous nonwoven sheet 1 is printed with a pattern 3 (a plurality of individually paper-wrapped candy balls) using the so-called electrophotographic printing process as will be described more in details. The pattern 3 is defined by toner 4 deposited on the fibrous nonwoven sheet.

The fibrous nonwoven sheet 1 comprises a fibrous nonwoven fabric 2 formed with a plurality of thermoplastic synthetic resin fibers and having a predetermined thickness. The nonwoven fabric 2 may be selected from those made by spun lace- and needle punch-processes in which the component fibers are mechanically entangled and those made by melt blown-, thermal bond-, spun bond-, chemical bond- and air through-processes in which the component fibers are hot welded or bonded by means of adhesive together at points of entanglement.

It is also possible to use composite a nonwoven fabric consisting of a melt blown nonwoven fabric having a high water-resistance and two layers of a spun bond nonwoven fabric having high strength and flexibility sandwiching the melt blown nonwoven fabric.

Component fiber of the nonwoven fabric may be selected from a group including polyolefine-, polyester- and polyamide-based fibers and polyethylene/polypropylene or polyethylene/polyester core-sheath type conjugated fiber and side-by-side-type conjugated fiber.

The nonwoven fabric 2 is preferably surface treated with corona discharge or plasma discharge to improve a wettability of the nonwoven fabric' surface to 40 dyn/cm or higher and thereby to improve a printability, or the surface of the nonwoven fabric 2 is preferably coated with resin primer in order to achieve such an improvement.

The toner 4 slightly permeates also into the nonwoven fabric 2 in a direction of its thickness through the surface thereof so that the toner 4 is partially deposited on fibers 2a lying on the surface of the nonwoven fabric 2 to define an outer layer 4a exposed on the surface of the nonwoven fabric 2 and partially deposited on fibers 2b lying in the interior of the nonwoven fabric 2 to define an inner layer 4b being contiguous to the outer layer 4a. Each pair of the individual fine toner portions 4 lying adjacent are not mixed together and these individual toner portions 4 are present in the nonwoven fabric 2 in the form of a plurality of dots.

While the toner 4 may slightly permeate the nonwoven fabric 2 through its surface, it never occurs that the toner 4 might permeate and spread throughout the nonwoven fabric 2. Thus, there occurs neither indistinctness of the pattern due to the toner 4 spreading nor unintentional variation in the color tone due to the toner portions 4 mixed together.

The outer layer 4a of the toner 4 has a thickness dimension in a range of 1 - 100µm. Strictly to describe, the outer layer 4a has its thickness dimension L1 in a range of 1 - µ. In the case of the outer layer 4a having its thickness dimension L1 less than 1µm, a desired diffused reflection of light on this outer layer 4a would become difficult and the color tone of the outer layer 4a would largely depend on the angle of sight. Variation of the color tone in the outer layer 4a may blur the surface color based on the diffused reflection of the light on the surface of the nonwoven fabric 2 and may lead to a corresponding indistinctness of the pattern 3. In the case of the outer layer 4a having its thickness dimension L1 exceeding µm, the outer layer 4a of the toner 4 would be readily collapsed depending on a fastness of the toner 4 and, if collapsed, the diffused reflection of the light in the outer layer 4a would become difficult. Like the case in which the outer layer 4a has its thickness dimension L1 less than 1 µ m.

A surface area of the outer layer 4a of the toner 4 exposed on the surface of the nonwoven fabric 2 is in a range of 10 - 100µm². With the surface area of the outer layer 4a being less than 10µm², the desired diffused reflection of the light on the outer layer 4a would become difficult and the surface color would be blurred to make the pattern 3 indistinct like the case in which the outer layer 4a has its thickness dimension L1 less than 1 *µ*m. With the surface area of the outer layer 4a exceeding 100*µ*m², the surface of the nonwoven fabric 2 would not be able to provide a resolution sufficient to represent details of the pattern 3 distinctly. It should be understood that the resolution on the surface of the nonwoven fabric 2 can be adjusted within a range of 400 x 400 dpi ~ 2,400 x 1,200 dpi.

A total thickness dimension L2 of the toner 4 inclusive of the outer layer 4a and the inner layer 4b is 10 *µ*m or larger and less than the thickness of the nonwoven fabric 2 itself. With the thickness L2 less than 10 µm, the desired diffused reflection of the light in the inner layer 4b would become difficult and, in consequence, the color tone of the inner layer 4b would be largely depend on the angle of sight. Variation of the color tone in the inner layer 4b would blur a transmitted color based on light absorption in the nonwoven fabric 2 and would make the pattern 3 indistinct.

The toner 4 may be liquid toner or powder toner. The liquid toner may be an appropriate mixture of binder resin, colorant to color the toner, insulating liquid comprising isoparaffin-based organic solvent, charge regulating agent serving to provide the toner with a polarity. The colorant may be selected from a group of various pigments such as carbon black, C.I.pigment Yellow 12, C.I.Pigment Red 48, C.I.Pigment Blue 15. For full color printing, color toners of yellow, magenta and cyan may be placed one upon another to express colorful effect.

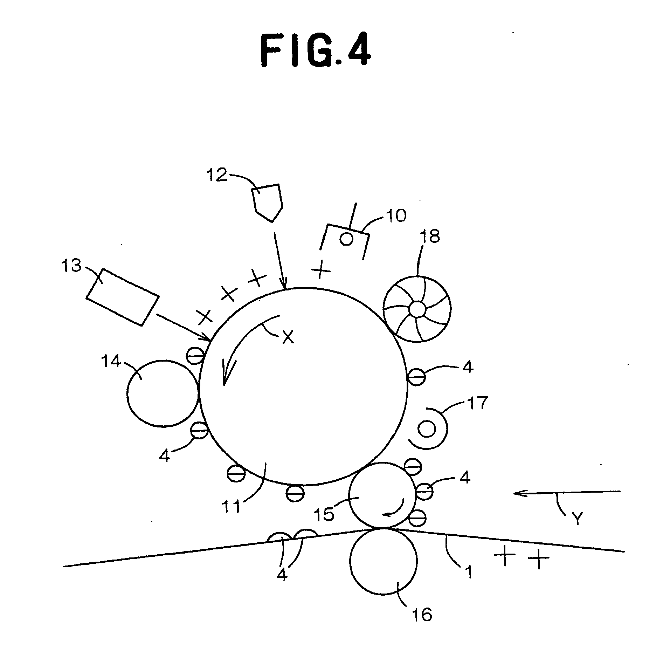

Fig. 4 is a diagram illustrating an example of electrophotographic printing process, in which a rotating direction of a photosensitive drum 11 is indicated by an arrow X and a travelling direction is indicated by an arrow Y. The electrophotographic process comprises the steps of charging, exposure, development, transfer and cleaning. Specifically, an electrostatic latent image is photoconductively formed on a peripheral surface of the photosensitive drum 11, the colored toner 4 (charged fine grains) is electrostatically deposited on the electrostatic latent image to convert the latent image to a visible image and the visible image formed by the toner 4 is transferred to the surface of the sheet member 1.

The step of charging comprises a step of uniformly charging the peripheral surface of the photosensitive drum 11 with positive ions using the corona discharge from an electrifying device 10. The step of exposure comprises a step of irradiating with the peripheral surface of the photosensitive drum 11 with the light rays emitted from an exposure device 12 and reflected on the original so that the charge on a region other than image region may be erased and the image region may remain charged (in the case of copying machine) or irradiating the image region with laser beam or LED beam from the exposure device 12 so that the charge on the image region may be erased (in the case of printer). The step of exposure forms on the peripheral surface of the photosensitive drum with the electrostatic latent image.

The step of development comprises a step of using a development device 13 to deposit the negatively charged toner 4 on the electrostatic latent image formed on the peripheral surface of the photosensitive drum 11 under the electrostatic effect to convert this electrostatic latent image to the visible image. Below the development device 13, there is provided a drum-like developer 14 in contact with the photosensitive drum 11.

The step of transfer comprises a step of transferring the visible image formed by the toner 4 to the fibrous nonwoven sheet 1 using a transfer roll 15 normally in contact with the peripheral surface of the photosensitive drum 11. This step is a so-called electrostatic transfer process, in which the fibrous nonwoven sheet 1 is electrified with the positive charge opposite to that of the toner 4 and the toner 4 is transferred from the peripheral surface of the transfer roll 15 to the fibrous nonwoven sheet 1. An impression drum 16 underlies the transfer roll 15 so that the impression drum 16 may press the fibrous nonwoven sheet 1 against the peripheral surface of the transfer roll 14.

The step of cleaning comprises a step of removing the residual toner 4 from the peripheral surface of the photosensitive drum 11. An example of this step is a so-called blade cleaning step comprising steps of erasing the charge on the peripheral surface of the photosensitive drum 11 by a charge eraser 17 and bringing a cleaner 18 having an urethane rubber blade in contact with the peripheral surface of the photosensitive drum 11.

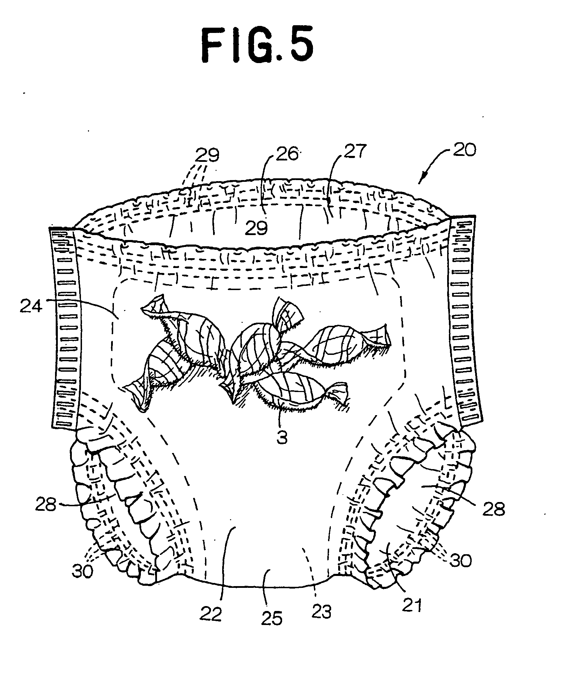

Fig. 5 is a perspective view of a disposable diaper 20 using the nonwoven fabric 2 printed with the pattern 3. The diaper 20 basically comprises a liquid-pervious topsheet 21, a liquid-impervious backsheet 22 and a liquid-absorbent core 23 disposed between the top- and backsheets 21, 22 and having its entire surface covered with and bonded to water-pervious tissue paper. The core 23 is bonded to at least one of the top- and backsheets 21, 22 with the tissue paper therebetween. This diaper 20 uses the nonwoven fabric 2 printed with the pattern 3 by the electrophotographic process as the backsheet 22.

The diaper 20 is composed, in the longitudinal direction, of a front waist region 24, a rear waist region 26 and a crotch region 25 extending between the front and rear waist regions 24, 26. The top- and backsheets 24, 26 have their transversely opposite side edge portions put flat and bonded to each other to define a waist-opening 27 and a pair of leg-openings 28.

An elastic member 29 comprising a plurality of elastic elements extending in a waist-surrounding direction are secured under tension to a peripheral edge portion of the waist-opening 27 so that this elastic member 29 is associated with the waist-opening 27. Similarly, elastic members 30 each comprising a plurality of elastic elements extending in a leg-surrounding direction are secured under tension to peripheral edges portions of the respective leg-openings 28 so that these elastic members 30 are associated with the leg-openings 28.

In this diaper 20, even when the backsheet 22 is curved as a wearer's body moves and, in consequence, an angle of sight with respect to the pattern 3 correspondingly varies, no change of color tone occurs in both the outer layer 4a and the inner layer 4b of the tone grains 4 so significant to make the pattern 3 indistinct. This is because a diffused reflection occurs in both the outer layer 4a and the inner layer 4b of the toner 4.

The topsheet 21 may be formed with the liquid-pervious sheet such as a hydrophilic nonwoven fabric or porous plastic film. The backsheet 22 may be formed with a hydrophobic nonwoven fabric or a laminated sheet consisting of the liquid-impervious plastic film placed upon the hydrophobic nonwoven fabric.

The core 23 is a mixture of fluff pulp, high absorption polymer particles and thermoplastic synthetic resin fiber compressed to a desired thickness. The high absorption polymer may be selected from a group including starch-, cellulose-based polymer and synthetic polymer.

Bonding of the top- and backsheets 21, 22 together, bonding of the core 23 and bonding of the elastic members 29, 30 may be carried out using a suitable adhesive agent such as a hot melt adhesive agent or a technique of welding such as a heat- or sonic-sealing.

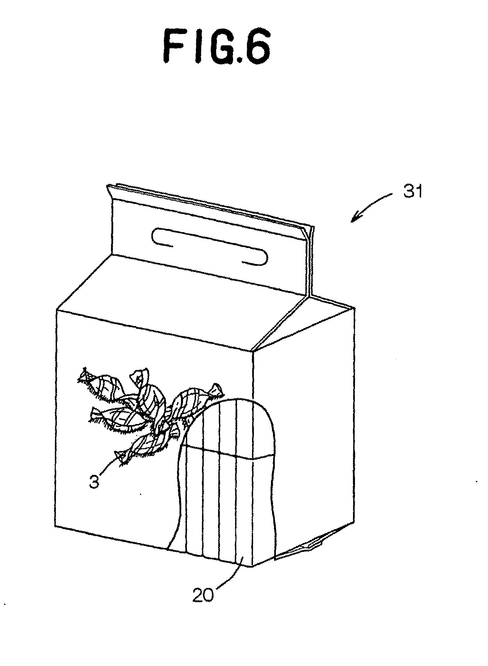

Fig. 6 is a perspective view of a package 30 using the nonwoven fabric 2 printed with the pattern 3 by the electrophotographic process. The package 30 has its side faces printed with the same patterns 3 as in the diaper 20 of Fig. 5. A plurality of the folded diapers 20 are packed within the package 30.

The fibrous nonwoven sheet 1 according to this invention is applicable not only to the disposable diaper but also to a other body fluid absorbent wearing article such as a diaper cover, a training pant, a pant for incontinent patient or a sanitary napkin.

The fibrous nonwoven sheet according to this invention is formed with a nonwoven fabric electrophotographically printed with a given pattern. While the toner may slightly permeate the nonwoven fabric, there is less likely that the toner might permeate the nonwoven fabric to its interior and spread therein. Therefore, it is not concerned that the toner might run so as to make the pattern indistinct and/or mingled together to cause unintentional variation of the color tone.

In the sheet member according to this invention, the outer layer of the toner 4 has its thickness dimension in a range of 1 - 100µm and has its surface area exposed on the surface of the nonwoven fabric in a range of 10 - 100 *µ*m². With such dimensioning, the desired diffused reflection of the light is ensured in the outer layer and the color tone in this outer layer is substantially free from any remarkable affection of a variation in the angle of sight. Additionally, the total thickness dimension of the toner inclusive of the outer and inner layers is 10µm or larger and less than the thickness of the nonwoven fabric itself. With such dimensioning, the desired diffused reflection of the light is ensured in the inner layer and the color tone is substantially not affected by a variation in the angle of sight. In this manner, the sheet member according to this invention allows the pattern to maintain its distinctness independently of a variation in the angle of sight.

With the disposable body fluid absorbent wearing article using the fibrous nonwoven sheet according to this invention as the backsheet, the desired diffused reflection of light occurs in both the outer layer and the inner layer of the toner even when the angle of sight with respect to the pattern varies as the backsheet is curved. Therefore, a difference in the color tone between the outer and inner layers is minimized. In other words, the pattern printed on the backsheet are distinctly seen independently of a variation in the angle of sight.

## Claims

1. A fibrous nonwoven sheet (1) printed with a given pattern (3) using an electrophotographic process comprising the steps of photoconductively forming an electrostatic latent image on a photosensitive body, electrostatically depositing colored toner (4) (charged fine grains) on said electrostatic latent image to convert said electrostatic latent image to the corresponding visible image and transferring said visible image onto a surface of said fibrous nonwoven sheet using an electrostatic transfer process, wherein:
said fibrous nonwoven sheet is made of a plurality of thermoplastic synthetic resin fibers and has a given thickness; said toner has an outer layer (4a) slightly permeating said fibrous nonwoven sheet through a surface thereof and deposited around fibers (2a) lying in a vicinity of said surface of said fibrous nonwoven sheet and an inner layer (4b) deposited around fibers (2b) immediately underlying said surface; the toner is present on the fibrous nonwoven sheet in the form of a plurality of independent dots and respective pairs of adjacent dots of the toner are not mingled together; the outer layer of each toner dot has a thickness dimension in a range of 1 to 100 µm; the outer layer of each toner dot exposed on said surface of said fibrous nonwoven sheet has a surface area in a range of 10 to 100 µm²; and a thickness dimension of each toner dot inclusive of said outer layer and said inner layer is 10 µm or larger and less than a thickness of said nonwoven sheet itself.

2. The fibrous nonwoven sheet according to Claim 1, wherein said fibrous nonwoven sheet is used as at least a liquid-impervious backsheet (22) in a disposable body fluid absorbent wearing article comprising a liquid-pervious topsheet (21), said liquid-impervious backsheet and a liquid-absorbent core (23) disposed between said top- and backsheets.

## Patentansprüche

1. Vlieslage (1), die mit einem vorgegebenen Muster (3) bedruckt ist, indem ein elektrophotographisches Verfahren mit Schritten zum photoleitfähigen Ausbilden eines elektrostatischen latenten Bilds auf einem photoempfindlichen Körper, elektrostatischen Aufbringen farbigen Toners (4) (geladener feiner Körner) auf dem elektrostatischen latenten Bild, um es in ein entsprechendes sichtbares Bild umzuwandeln, und Übertragen des sichtbaren Bilds auf eine Fläche der Vlieslage unter Verwendung eines elektrostatischen Transferverfahrens verwendet wird, wobei
die Vlieslage aus mehreren thermoplastischen Kunstharzfasern hergestellt ist und eine gegebene Dicke aufweist, der Toner eine Außenschicht (4a), die durch eine Oberfläche der Vlieslage etwas in diese eindringt und sich um in der Nähe der Oberfläche der Vlieslage liegende Fasern (2a) herum ablagert, und eine Innenschicht (4b) aufweist, die sich um unmittelbar unter der Oberfläche liegende Fasern (2b) herum ablagert, der Toner auf der Vlieslage in Form mehrerer unabhängiger Punkte vorliegt und entsprechende Paare benachbarter Punkte des Toners nicht miteinander vermischt sind, die Außenschicht jedes Tonerpunkts ein Dickenmaß im Bereich von 1 bis 100 µm aufweist, die Außenschicht jedes Tonerpunkts, der auf der Oberfläche der Vlieslage freiliegt, eine Oberflächengröße im Bereich von 10 bis 100 µm² aufweist und das Dickenmaß jedes Tonerpunkts einschließlich der Außenschicht und der Innenschicht 10 µm oder mehr, jedoch weniger als die Dicke der Vlieslage selbst beträgt.

2. Vlieslage nach Anspruch 1, die zumindest als flüssigkeitsundurchlässige rückwärtige Lage (22) in einem körperflüssigkeitsabsorbierenden Wegwerfkleidungsstück mit einer flüssigkeitsdurchlässigen oberen Lage (21), der flüssigkeitsundurchlässigen rückwärtigen Lage und einem zwischen der oberen und der rückwärtigen Lage angeordneten absorbierenden Kern (23) verwendet wird.

## Revendications

1. Feuille de non-tissé fibreux (1) imprimée avec un certain motif (3) en utilisant un procédé électrophotographique comprenant les étapes consistant à former par photoconduction une image latente électrostatique sur un corps photosensible, déposer par voie électrostatique du toner coloré (4) (grains fins chargés) sur ladite image latente électrostatique afin de convertir ladite image latente électrostatique en l'image visible correspondante, et transférer ladite image visible sur une surface de ladite feuille de non-tissé fibreux en utilisant un procédé de transfert électrostatique, dans laquelle :
ladite feuille de non-tissé fibreux est constituée d'une pluralité de fibres de résine synthétique thermoplastique et a une épaisseur donnée ; ledit toner présente une couche extérieure (4a) passant légèrement au travers de ladite feuille de non-tissé fibreux par une surface de celle-ci et déposée autour de fibres (2a) s'étendant au voisinage de ladite surface de ladite feuille de non-tissé fibreux, et une couche intérieure (4b) déposée autour de fibres (2b) se trouvant directement au-dessous de ladite surface ; le toner est présent sur la feuille de non-tissé fibreux sous la forme d'une pluralité de points indépendants, et des paires respectives de points adjacents du toner ne sont pas mélangées l'une à l'autre ; la couche extérieure de chaque point de toner a une dimension dans le sens de l'épaisseur se situant dans la gamme de 1 à 100 µm; la couche extérieure de chaque point de toner, exposé sur ladite surface de ladite feuille de non-tissé fibreux, a une aire se situant dans la gamme de 10 à 100 µm² ; et la dimension dans le sens de l'épaisseur de chaque point de toner, en incluant ladite couche extérieure et ladite couche intérieure, est de 10 µm ou plus, et est inférieure à l'épaisseur de ladite feuille de non-tissé elle-même.

2. Feuille de non-tissé fibreux selon la revendication 1, dans laquelle ladite feuille de non-tissé fibreux est utilisée au moins comme une feuille arrière (22) imperméable aux liquides dans un article vestimentaire jetable absorbant les fluides corporels, et comprenant ensemble une feuille supérieure (21) perméable aux liquides, ladite feuille arrière imperméable aux liquides, et un noyau central (23) absorbant les liquides et disposé entre lesdites feuilles supérieure et arrière.
